# EUROPEAN PATENT SPECIFICATION

(11) **EP 4 168 388 B1**
(45) Date of publication and mention of the grant of the patent: **05.08.2026**
(21) Application number: 21749690.0
(22) Date of filing: 18.06.2021
(51) Int. Cl.: C07C 227/40, C07C 229/16, C11D 3/33

(54) **PROCESS TO PREPARE A CO GRANULE OF METHYLGLYCINE N,N DIACETIC ACID SALTS EMPLOYING A CRUMBLY PHASE COMPOSITION OF METHYLGLYCINE N,N DIACETIC ACID SALTS**
VERFAHREN ZUR HERSTELLUNG EINES CO-GRANULATS VON METHYLGLYCIN-N,N-DIESSIGSÄURESALZEN UNTER VERWENDUNG EINER KRÜMELPHASENZUSAMMENSETZUNG VON METHYLGLYCIN-N,N-DIESSIGSÄURESALZEN
PROCÉDÉ DE PRÉPARATION D'UN CO-GRANULÉ DE SELS D'ACIDE MÉTHYLGLYCINE-N,N-DIACÉTIQUE UTILISANT UNE COMPOSITION À PHASE FRIABLE DE SELS D'ACIDE MÉTHYLGLYCINE-N,N-DIACÉTIQUE

(30) Priority: 19.06.2020 EP 20181237
(43) Date of publication of application: 26.04.2023
(73) Proprietor: Nouryon Chemicals International B.V., 6824 BM Arnhem (NL)
(72) Inventor: VAN HAEREN, Paulus Johannes Cornelis, 7418 AJ Deventer (NL); HEUS, Martin, 7418 AJ Deventer (NL)
(74) Representative: Ingrassia, Fisher & Lorenz UK Ltd.
(86) International application number: PCT/IB2021/000431
(87) International publication number: WO 2021/255525

(56) References cited:
- EP-A1- 2 392 638
- WO-A1-2019/007944
- WO-A1-2020/064379
- US-A1- 2018 355 285
- US-A1- 2020 181 537

## Description

The present application claims priority of European Patent Application No. EP20181237.7, filed June 19, 2020.

The present invention relates to employing a crumbly phase composition of MGDA-Nax, sodium salts of methylglycine N,N diacetic acid, to give a crystalline co granule MGDA-Nax product.

MGDA-Nax (which actually is MGDA-NaₓZ₃₋ₓ, Z commonly being H, for sake of easy reference referred to as MGDA-Nax in this document), wherein x is 2.5 to 3, the sodium salt of methylglycine-N,N-diacetic acid, is a known chelating agent having a good biodegradability, and is employed in several applications. Many of these applications involve the use of solid, preferably granular, MGDA-Nax. For example, when formulating solid detergent compositions such as powdery or tableted dishwashing formulations it is important that the MGDA-Nax component is available in a dry and solid form.

Preparing a solid of MGDA-Nax is not always straightforward. Storing a MGDA-Nax solid similarly also has its challenges. If the solid MGDA-Nax is mainly amorphous it will be quite hygroscopic, and hence sensitive to storage at humid conditions, in which case the material absorbs water, yielding a tacky material which makes the solid less suitable for use in solid formulations as these solids quickly lose their free-flowing properties.

The hygroscopicity of solid MGDA-Nax was found to be lower when the MGDA-Nax is present in (primarily the) crystalline form. When isolated as a crystal instead of as an amorphous solid, the free-flowing properties of MGDA are also improved. Some varieties of crystalline MGDA-Na3 (the trisodium salt of methyl glycine-N,N-diacetic acid) are known in the art, recognizable via XRD-analysis, yielding different characteristic diffraction patterns.

Today three crystalline modifications are known for MGDA-Na3.

WO2012/168739 discloses a process of spray drying MGDA-Na3 starting from a slurry, next agglomerating the obtained solid and subsequently comminuting the obtained agglomerate. The document says that using this process more of the crystalline dihydrate is obtained over the less desired monohydrate. The dihydrate crystal in this document will be referred to as crystal type I and what is called the monohydrate is referred to as crystal type II.

WO2019/007944 discloses a third crystal type, called crystal type III.

The crystal types I, II and III can be defined by the below diffraction patterns as given in Table 1.

**Table 1 Crystal Type I, II and III diffraction patterns**

| type I | | type II | | Type III | |
|---|---|---|---|---|---|
| 2Θ | d (Å) | 2Θ | d (Å) | 2Θ | d (Å) |
| 8.2 | 10.8 | 8.4 | 10.5 | 5.8 | 15.2 |
| 10.5 | 8.4 | 9.5 | 9.3 | 7.5 | 11.8 |
| 15.6 | 5.7 | 11.1 | 8 | 8.1 | 10.9 |
| 16.5 | 5.4 | 13.2 | 6.7 | 9.5 | 9.3 |
| 17.1 | 5.2 | 13.9 | 6.4 | 11.7 | 7.6 |
| 18.1 | 4.9 | 15.8 | 5.6 | 13.9 | 6.4 |
| 18.8 | 4.7 | 16.5 | 5.35 | 15.1 | 5.9 |
| 21 | 4.25 | 16.8 | 5.25 | 16.5 | 5.4 |
| 21.4 | 4.15 | 17.3 | 5.1 | 17.3 | 5.1 |
| 22.6 | 3.9 | 17.7 | 5 | 18.5 | 4.8 |
| 23.7 | 3.75 | 18.9 | 4.7 | 19.1 | 4.65 |
| 24.7 | 3.6 | 20.3 | 4.35 | 20.1 | 4.4 |

In WO2012/168739 it is shown that for many applications crystal type I is the preferred variety, as it is less hygroscopic than crystal type II. Powders or granules containing a high degree of crystal type I keep their free-flowing character better upon storage at high humidity conditions, while products containing only or mainly the type II variety fail at these conditions.

The properties of solid MGDA-Nax products can be further improved if a co granule of MGDA is made that contains a second component.

Co granules of MGDA-Nax are disclosed in several documents such as EP2726442. They are prepared by mixing dry MGDA-Na3 and silica in a tumble mixer

WO 2010/133618 discloses a process of concentrating an aqueous solution of MGDA-Na3 (20-60 wt%) in an evaporator with rotating internals yielding a crystal slurry having a solid concentration in the range from 60 to 85 wt%, which is subsequently aged in a paste bunker and then dosed to a thin-film contact dryer. Two different crystal varieties or mixtures of these can be obtained by this process, referred to as crystal modification 1 and 2, corresponding to types II and I respectively in table 1. Even though WO 2010/133618 specifies the solid concentration to be 60 to 85 wt% it also clearly states that this composition is a slurry. The Examples of WO 2010/133618 do not go higher than slurries containing 69 wt% of solids, which are cooled while stirring, from which it must be concluded that one still deals with a liquid dispersion, and not a crumbly phase composition. The process of WO 2010/133618 is rather sensitive towards concentration fluctuations as in the concentration ranges as mentioned in the examples, MGDA-Na3 behaves as a thixotropic paste, which rheological properties are highly dependent on concentration, seriously raising the chance on fouling, in case of process parameter fluctuations, up to full blockage of the process line.

WO2018/153876 discloses a process to crystallize MGDA alkali metal salts in the presence of a particulate solid with a specific pore volume such as ground alumina, ground molecular sieves or silica powder in an amount of 0.1-2.0 wt% on the basis of a 35-60wt% MGDA alkali metal salt solution. The process leads to solid MGDA alkali metal products with high crystallinity that contain at least 90 wt% of crystal type I and at least 1 wt% of crystal type II.

WO 2015/173157 discloses a process to crystallize chelating agents from a dispersion wherein the dispersion is milled. In the Examples also MGDA-Na3 is crystallized employing the above process. In the Example wherein MGDA-Na3 is crystallized, to a dispersion that contains 50 wt% of MGDA-Na3, 20wt% of MGDA-Na3 seeds are added, to give a dispersion hence containing 58wt% of MGDA-Na3. Following the above process, a product is obtained that has a crystallinity of 67% when milling is employed and 60% when in comparison no milling is employed. The process is further characterized in that it concerns the presence of a considerable amount of water.

WO2011/023382 and WO2009/103822 disclose processes for preparing MGDA-Na3 by spray granulating an aqueous solution or slurry of MGDA-Na3. Important disadvantages of such processes is that the energy consumption of such process is high and that equipment for spray granulation requires quite a lot of factory space. Moreover, using these processes, it is very difficult up to impossible to obtain a product having a high crystallinity, in particular when one aims after obtaining a product containing for example only the favored type I crystalline variety.

WO2017/102483 discloses a process to crystallize MGDA-Na3 by salting out. Such a process will lead to a product that is contaminated with salt impurities, unless the product is washed extensively, yielding a waste stream.

A general disadvantage of crystallization processes in which the crystals are harvested, which also holds for WO2012/150155 disclosing a seeded evaporative crystallization process of L-MGDA-Na3 or WO2015/173157, is that one ends up with a mother liquor, in which the byproducts of the MGDA-Na3 production process are concentrated, eventually yielding a waste stream. Also, crystallization processes for drying MGDA-Na3 usually involve the circulation of considerable amounts of water as a solvent or as a mother liquor, or when the mother liquor is disposed of, the creating of considerable waste streams.

WO 2020/064379 A1 discloses a process for making a granule containing a chelating agent (such as MGDA-Nax) and an alkali metal salt of a fatty acid by mixing the components in the presence of water and removing most of the water by spray-granulation or spray-drying.

US 2018/355285 A1 discloses a process for manufacturing granules of aminopolycarboxylic acid salts (such as MGDA-Na3) by spray-granulating an aqueous slurry which may optionally also comprise silica, a silicate or organic polymers.

US 2020/181537 A1 discloses a process for manufacturing granules of salts of a mixture of MGDA and glutamic acid diacetic acid (GLDA) by spray-granulating an aqueous solution of the mixed salts, optionally in the presence of silica, a silicate or organic polymers.

The present invention aims to provide an improved process to prepare solid crystalline co granules of crystalline MGDA-Nax that does not have the above disadvantages. The invention is based on a special form of MGDA-Nax, called the crumbly phase, in which there is limited water and the MGDA-Nax is present in a crystalline state for the major part so that a non-pasty, crumble like texture is obtained. When such crumbly phase is used as a feed for a drying process, one obtains an efficient process characterized by a high yield of high-quality product, in which circulating water can be limited, and waste streams can be avoided. The co granules as obtained by the process are characterized as very easy to dry, and have a good porosity and improved bulk density, storage stability, and hygroscopicity (reduced moisture sensitivity),

The present invention provides a process to prepare solid crystalline co granules of MGDA-Nax, x being 2.5-3, comprising a step of drying a MGDA-Nax-containing crumbly phase composition containing on total weight of the composition
(i) 70 - 87 wt% of organic compounds and salts thereof wherein 85 to 100 wt% on total organic compounds and salts thereof is MGDA-Na3 and at least 60 wt% of the MGDA-Na3 is crystalline, and
(ii) 13 - 30 wt% of water

In the presence of a second composition that contains at least one second component selected from the group of scale inhibitors, crystal inhibitors, film or spot preventing polymers, glass-corrosion inhibiting agents, pH modifiers, chelating agents, builders, bleaching agents, and surfactants.

The crumbly phase is a phase comprising weakly agglomerated particulate solids covered with a thin layer of an aqueous composition, such as of a MGDA-Nax composition, showing a rheological behavior that resembles or at least approaches the behavior of dry particulate material. The thin layer of the MGDA-Nax composition is preferably a (saturated) aqueous solution of MGDA-Nax.

It should be noted that though the crumbly phase product is defined as having an organic compounds and salts content and a water content, this is not intended to mean that the water is fully present as a separate liquid water phase. Part of the water can be present as crystal water and thereby can be seen as solid-state water. In the crumbly phase composition as covered by the present invention the water is defined to cover both free water and crystal water. The amount of water can be determined by Karl-Fischer titration.

Crumbly phase behavior has the advantage compared to a thixotropic paste of much easier handling and was found to be much easier dried into free-flowing granules.

Organic compounds are compounds that have a hydrocarbon backbone wherein this hydrocarbon backbone may contain one or more heteroatoms like oxygen or nitrogen atoms. The group of organic compounds includes organic acids and bases such as carboxylic acids. If carboxylic acids are present in the organic compounds fraction, the salts of such carboxylic acids are also seen as part of the organic compounds fraction in this document. Crystal water is not included in the weight fraction of the organic compounds, and neither are inorganic compounds such as inorganic salts.

It may be noted that EP0845456 also relates to a process of crystallizing a composition of MGDA-Na3 with a limited amount, namely 10-30%, of water. However as is clear from the Examples in this document, the starting composition employed in this document contains merely amorphous MGDA-Na3. To prepare a MGDA-Na3 solid of high crystalline content, mechanical stress is employed during the drying process. During crystallization a pasty, highly thixotropic, intermediate phase is formed. This gives rise to an increased chance on product quality fluctuations in case of process parameter fluctuations. It also seriously enhances the chance on full blockage of the process line, especially in case of power interruptions and the formation of a MGDA-Na3 solid product that is one clump, in the worst case of a concrete-like or gum-like texture that cannot be granulated anymore after it has been dried by commonly available mechanical granulation means such as a mill.

The present invention, identifying and exploiting a phase (assigned; crumbly phase) other than the paste phase, in which the composition shows granular flow behavior, avoids the problems that are related to the process described in EP0845456 and will effectively yield crystalline co granules of MGDA-Nax

Using the process of the present invention wherein the above crumbly phase composition is subjected to a drying step in the presence of the second composition, surprisingly a co granule of MGDA-Nax crystalline product is obtained that has a high crystallinity, high bulk density, with predominantly or only close to spheroidal shaped particles having the particle size as requested by e.g. the detergent industry which is one of the main outlets for MGDA-Nax. The crystalline MGDA-Nax as obtained was determined to consist for the major part of a particular crystal type, such as crystal type I if the crumbly phase product employed in preparing it also contains predominantly this same crystal type, such as the favored type I. The crystalline product remains free flowing at 70% relative humidity (RH) and 40°C for at least 144 hours.

In addition, the process is a highly efficient process characterized by a high yield of high-quality product and no waste streams.

In the process of the invention the second composition is preferably added to the crumbly phase composition of MGDA-Nax, or alternatively, during the preparation of this crumbly phase, as a solid composition or an aqueous solution. Even more preferred the second composition is added as an aqueous solution that is highly concentrated, saturated or supersaturated, or dosed in several steps or continuously over a period of time, to continue to maintain the combined MGDA-Nax plus second component-containing composition in a crumbly state. If the second composition is added during the preparation of the crumbly phase of MGDA-Nax, in embodiments the process contains as steps adding together solid MGDA-Nax, a solution of MGDA-Nax and the second composition, wherein the solid MGDA-Nax and solution of MGDA-Nax are mixed such that a crumbly phase MGDA-Nax is acquired, and preferably the second composition is of a nature that the crumbly state is maintained during the steps of adding together the several components.

The second composition contains at least one second component selected from the group of scale inhibitors, crystal inhibitors (e.g. phosphonates), film or spot preventing polymers, glass-corrosion inhibiting agents (e.g. Zn, Bi salts), pH modifiers (sodium carbonate / bicarbonate), chelating agents, builders, bleaching agents, and surfactants. Preferably the second component is a glass-corrosion inhibitor such as a silica or a silicate salt, a zinc salt, or bismuth salt; a scale inhibiting polymer such as an acrylate polymer, or a sulfopolymer based on ethylenically unsaturated monomers that contain a sulfonate or sulfonic acid function, a crystal inhibiting polymer, a film or spot preventing polymer, or a chelating agent such as citric acid

MGDA-Nax is a sodium salt of MGDA wherein 2.5 to 3 molar equivalents of sodium are present (x is 2.5-3) on 1 MGDA molar equivalent. Preferably, x is 2.7-3, most preferably x is about 3.

Finally, the present invention provides the product obtainable by the drying process.

In embodiments, the drying process delivers solid crystalline co granules of MGDA-Nax product that contains between 50 and 80 % of the L enantiomeric form of MGDA (and hence 20-50% of the D enantiomeric form of MGDA) and that contains 90-99% MGDA-Nax of crystal type I and 1-10% MGDA-Nax of crystal type III on the basis of the total amount of MGDA-Nax crystals in the MGDA-Nax co granule product, wherein x is preferably 3.

Preferably, the solid crystalline co granule MGDA-Nax product obtainable by the drying process has a MGDA crystallinity between 60 and 100%, more preferably between 70 and 100%, most preferably between 75 and 100%.

In another preferred embodiment the co granule MGDA-Nax product contains on total co granule weight between 40 and 89 wt% of MGDA-Nax and between 0.5 and 40wt% of the second component(s), more preferably 77 to 89 wt% MGDA-Nax and 0.5 to 10 wt% of the second component(s), most preferably 82 to 89 wt% of MGDA-Nax and 0.2 to 5wt% of the second component(s). The balance contains water, and in embodiments, organic compounds other than MGDA, and inorganic salts other than second component.

In another preferred embodiment the MGDA-Nax crumbly phase composition and solid crystalline MGDA-Nax product obtainable by the drying process contains less than 0.01 wt% particulate solid with a pore volume in the range of from 0.25 to 0.75 cm³/g, determined by nitrogen adsorption in accordance with 66134:1998-02 on total MGDA-Nax weight.

In yet another preferred embodiment the solid crystalline co granules of MGDA-Nax product obtainable by the drying process contains 92-97% MGDA-Nax of crystal type I and 3-8% MGDA-Nax of crystal type III on the basis of the total amount of MGDA-Nax crystals in the product, wherein more preferably x is 3.

In yet another preferred embodiment the MGDA-Nax crumbly phase composition contains between 20 and 30 wt% of water, more preferred between 21 and 27 wt% of water, most preferred between 22 and 26 wt% of water, the wt% being based on the total weight of the crumbly phase composition.

The weight percentage of organic compounds and salts thereof in the crumbly phase composition is in a preferred embodiment between 70 and 80 wt%, more preferred between 73 and 79 wt%, most preferred between 75 and 78 wt%, the wt% being based on the total weight of the crumbly phase composition.

Of the organic compounds and salts thereof as said at least 85 wt% is MGDA-Nax. Preferably at least 90 wt% of the organic compounds and salts thereof is MGDA-Nax. Other organic compounds and salts thereof that may be present include compounds that can be found in MGDA as remainders of the production process due to e.g. incomplete reaction of starting materials, side products, or compounds that are purposively added as an additive and include compounds such as citric acid or citrate salts, glycolic acid or glycolate salts, NTA-Nax, formic acid or formate salts, wherein NTA-Nax stands for the sodium salt of nitrilotriacetic acid.

In the water fraction in the crumbly phase a trace amount of inorganic salts can be present. Such salts may be sodium hydroxide, or sodium chloride.

In a preferred embodiment the particles to make the crumbly phase have a crystallinity of at least 60%, more preferred at least 70%, most preferred at least 75% and up to and including 100%. The particles in an embodiment contain crystal type I, or at least 75% of the crystalline MGDA-Nax in the particles is of crystal type I, more preferably at least 90%.

In another preferred embodiment the MGDA-Nax in the crumbly phase composition and in the obtained co-granule is at least 70% crystalline, even more preferably at least 75%.

The MGDA-Nax in the crumbly phase may be of the L enantiomeric form or of the D enantiomeric form. Preferably, the MGDA-Nax is 50-100% in the L enantiomeric form (and thus 0-50% in the D enantiomeric form). Even more preferably the MGDA-Nax is 50-80% in the L enantiomeric form, most preferably 50-65% in the L enantiomeric form.

The solid crystalline co granules of MGDA-Nax product obtainable by the drying process in a preferred embodiment also is 50-100% in the L enantiomeric form (and thus 0-50% in the D enantiomeric form), even more preferably 50-80% in the L enantiomeric form, most preferably 50-65% in the L enantiomeric form.

It should be noted that the above three crystal types I, II and III have been primarily observed for MGDA-Na3 wherein there is a detectable amount of D enantiomer.

In an embodiment the process to dry the crumbly phase composition to prepare the crystalline co granule of MGDA-Nax involves the dosage of the second composition to the crumbly MGDA phase. The second composition is dosed either as an aqueous liquid, highly concentrated, saturated or supersaturated or as a slurry via e.g. a nozzle or dosing pump, or alternatively the second composition can be dosed as a solid by a dosing feeder to a granulator/ mixer e.g. Lodige mixer, Duplex mixer. The dosing of the second composition can be done in various ways but it should preferably be done in such a way that the amount of water added together with the second composition does not change the physical handling of the mixture of MGDA-Nax and second composition, preferably the crumble phase characteristics are maintained. Preferably, after the compounds are thoroughly mixed, the mixture is submitted to a drying step, a step selected from the group of an evaporation step, a step of fluid bed drying, a step of thin film drying, and, a step of drum drying, and a spray granulation step. The composition is in particular efficiently dried by simple drying processes such as drying processes that are based on simply evaporating free water such as in an oven, in a rotating evaporator, a drum a thin film drier, or on a moving belt or a fluid bed. When such processes involve a step in which the composition of MGDA-Nax and second composition is subjected to some mechanical energy, like in a fluid bed, a rotating drum, or on a moving belt, the co granule product is immediately available in granules, when the drying step is done in the absence of mechanical energy such as in an oven, the product is obtained as a porous cake of solid crystalline material which has been found to be extremely easily granulated.

In the drying process a part of the crystalline product as obtained can be recycled into the process and mixed with at least one of an aqueous solution containing MGDA-Nax, additional crumbly phase of MGDA-Nax or second composition.

The drying step in a preferred embodiment is performed for a residence time of 1 minute to 5 hours if performed in an oven. When the drying step is done using a fluid bed, a thin film drier, a drum dryer or a moving belt, the drying step is preferably performed for a time of between 10 seconds and 30 minutes, even more preferably between 30 seconds and 15 minutes.

The drying step in another preferred embodiment is performed at a temperature of between 30 and 300 °C. When an oven is used the temperature is more preferably between 40 and 100°C, while using a fluid bed, a drum or another rotating evaporator, or a moving belt the drying temperature is in more preferred embodiments between 70 and 200°C.

The drying process may be done as a batch process, semi continuous or continuous process. Preferably the process is performed continuously.

To identify the crystal type varieties and to determine the crystallinity, diffractograms were recorded using a Bruker-AXS D8 reflection-diffractometer with Ni filtered Cu-Kα radiation. Generator settings are 40 kV, 40 mA. Fixed sample irradiation 15 mm, Soller slits 2.5 °. Measuring range: 2θ = 5.0 - 70.0°, step size 0.02°, time per step 0.25 seconds.

The degree of crystallinity was ascertained from the X-ray powder diffractograms by determining the surface fraction of the crystalline phase and of the amorphous phase and using these to calculate the degree of crystallinity (also called "crystallinity"), as the ratio of the area of the crystalline phase, Ic, to the total area, consisting of the area of the amorphous phase, la, and the area of the crystalline phase, crystallinity(%): Ic/(Ic+la)*100.

This procedure was performed using Bruker EVA v.4.2.1.10 software with the following parameters: enhancement disabled, curvature 1, threshold 1.

Where in this document is referred to "free-flowability", this property was judged qualitatively by the following method

About 4 grams of the materials were weighted into a crystallization dish (10 cm diameter) and evenly distributed over the bottom and subsequently stored in in a calibrated 70% RH climate chamber at 40 °C (Weiss SB11 500)

After storage the dish was tilted about 60° and gently tapped.
- When all material falls to one side the material is judged "free flowing";
- when a significant part of the material remains sticking on the bottom of the dish, the material is judged "partly free flowing";
- when all material is stuck to each other the material is judged "caked".
- A fourth option is that the material is partly up to fully "dissolved"; initially to be recognized by the appearance of a liquid phase, either in the form of tiny droplets on the glass walls or a glassy shiny layer upon the particle bed.

Bulk density was determined as freely settled bulk density.

The invention is illustrated by the examples below

### Examples

### Example 1 (comparative)

A 50 wt% MGDA-Na3 aqueous slurry was prepared by crystallization evaporation, using MGDA-Na3 seeds of the type I variety. To 100 ml of the slurry, 10 ml of 37 wt% sodium silicate in water was added. The slurry obtained was concentrated further using a laboratory rotary evaporator (temperature 50°C, pressure 200 mbar). At a concentration of 63 wt% MGDA-Na3, the slurry transformed into a pasty phase, yielding a sticky film on the wall of flask. Further drying yielded a hard film on the wall that could not be harvested efficiently anymore.

### Example 2 (comparative)

77 kg of a 40.7 wt% MGDA-Na3 aqueous solution was charged to a 80 L Vrieco-Nauta conical screw mixer. The solution was concentrated to 50wt% MGDA-Na3 (jacket temperature: 50-120°C, pressure 100-200 mbar, screw speed 70 rpm). After adding 300 grams of MGDA-Na3 seeds of the type I variety, 1 kg of a 37 wt% sodium silicate solution in water was added, and the slurry obtained was concentrated further. In the range of 60-65 wt% MGDA-Na3 a thixotropic pasty phase was formed, yielding serious fouling problems. The product eventually obtained was one large spheroidal lump of material that could not be processed further.

### Example 3 (comparative)

328.1 gram of a 40.2wt% MGDA-Na3 solution was concentrated by water evaporation under reduced pressure (at about 50mbar) in a rotary film evaporator (bath temperature 68°C) up to a concentration of 60.7wt% MGDA. The over-saturated MGDA was transferred into a plastic dish and immediately 23.9 gram of sodium silicate solution (ex Sigma Aldrich, containing ≥27% SiO2) was added and mixed thoroughly using a spatula. On addition of the sodium silicate solution, a jelly phase was formed, instantly. The mixture was seeded with 2.5 grams of solid powdery MGDA of the crystal type I, enforcing crystallization. The seeded mixture was dried over weekend in a circulation oven set at 80°C. The rather rigid solid product had to be crushed and milled using an impact miller equipped with a 20 mesh classification sieve in order to deliver a product that could be handled. XRD analysis of the powdery sample revealed that the product was crystalline (73% crystallinity) and consisted of solely type I crystals. Elemental analysis (ICP-OES) however revealed the presence of 1.4 wt% Si.

### Example 4 (according to the invention)

A crumbly phase composition was prepared by mixing 138.8 grams of solid MGDA-Na3, containing solely the type I crystal variety (MGDA-Na3 content 80wt%, crystallinity 75%) with 11.3 gram 40 wt% MGDA-Na3 aqueous solution with a spatula. The MGDA-Na3 solution was added in small portions of about 2ml allowing proper homogenization of the aqueous phase over the solid phase. The crystalline MGDA was agglomerated during the process. After addition of the aqueous MGDA-Na3, 8 grams of sodium silicate aqueous solution (ex Sigma Aldrich, containing ≥27% SiO2) was added to the crumbly phase, again in small portions of 2 ml while homogenizing the mixture with a spatula. The obtained composition was dried in a petri-dish overnight in an oven set at 90°C. the obtained co-granule was slightly caked but could be easily crumbled, yielding particles in the range of 0.2-5 mm. The co-granule showing a MGDA content of 78 wt% and a Si content of 0.7 wt%, appeared to be highly crystalline (80%) showing the Type I variety with a few (less then 3%) type III crystals.

### Example 5 (according to the invention)

A crumbly phase composition was prepared by mixing 17 grams of solid MGDA-Na3, containing solely the type I crystal variety (MGDA-Na3 content 80wt%, crystallinity 75%) with 3 gram 41 wt% MGDA-Na3 aqueous solution with a spatula. The MGDA-Na3 solution was added in small portions of about 1ml allowing proper homogenization of the aqueous phase over the solid phase. The crystalline MGDA was agglomerated during the process.

Subsequently, 17 grams spray dried Berol 08 (commercially available surfactant of Nouryon) was added to the crumbly phase and the mixture was homogenized with a spatula. After addition of the Berol 08, 6 grams 41wt% MGDA-Na3 solution was added, again in portions of 1 ml, while homogenizing the mixture with a spatula. The obtained composition was dried in a petri-dish overnight in an oven set at 50°C. The co-granule appeared to be highly crystalline (60%) showing Type I crystal variety (>99%).

### Example 6 (according to the invention)

The procedure of Example 4 was repeated but instead of 8 grams sodium silicate solution, 1.4 grams Zn sulfate monohydrate (glass corrosion inhibitor) was added in 4 fractions using a spatula resulting in a MGDA co-granule consisting of MGDA containing 0.4wt% Zn.

### Example 7 (according to the invention)

A crumbly phase composition of 250 grams solid MGDA-Na3, containing solely the type I crystal variety (MGDA-Na3 content 76.5wt%, crystallinity 75%) was dosed to a Duplex mixer (300ml), heated by a water bath up to 75°C and operated at a rotation speed of 40 RPM. 27 grams of sodium silicate solution (ex Sigma Aldrich, containing >27% SiO2) was added via a nozzle over a period of about 10 minutes. The mixture started to agglomerate. After dosing the resulting composition was discharged and dried at 75°C for 1 hour in an oven. The agglomerates were easily to crush and after sieving a free flowing MGDA co-granule of a very uniform particle size was obtained.

### Example 8 (according to the invention)

A crumbly phase composition was prepared by mixing 150 grams solid MGDA-Na3, containing solely the type I crystal variety (MGDA-Na3 content 80%wt%, crystallinity 75%) with 15 gram 41 wt% MGDA-Na3 aqueous solution in a Duplex mixer (300ml), operated at a rotation speed of 40 RPM. The MGDA-Na3 solution was added via a nozzle over a period of about 5 minutes. The mixture started to agglomerate.

To the crumbly phase 15.3 grams spray dried Alcoguard H5941 was added. After homogenization, 15 gram Alcoguard H5941 40wt% aqueous solution (commercially available readily biodegradable hybrid polymer of Nouryon) was dosed via a nozzle over a period of about 5 minutes. After dosing of the Alcoguard H5941 solution, the composition was mixed for 2 more minutes after which the resulting composition was discharged from the Duplex mixer and dried at 50°C overnight in an oven. The agglomerates were easily to crush while the product revealed a crystallinity of 51% containing Type I crystal variety (>99%).

### Example 9 (according to the invention)

50 gram of the composition as discharged from the mixer in Example 7 was dried in a fluid bed dryer (5 L table dryer) at 80°C for 8 minutes, yielding free flowing particles in the range of 0.1-5 mm, showing an MGDA-Na3 content of 83.5wt%. The crystallinity of the product was 83%, showing predominantly the type I variety and a few percent of the type III variety.

### Example 10 (according to the invention)

A mixture of a crumbly phase of 13.6 kg of solid MGDA-Na3, containing solely the type I crystal variety (MGDA-Na3 content 82wt%, crystallinity 75%) and 2.4 kg of solid sodium silicate penta hydrate were added to a 50 L Lödige Ploughshare mixer, operated at a rotation speed of 170 rpm, and, subsequently, 1.5 kg of a 40.7 wt% MGDA-Na3 aqueous solution was dosed, during 8 minutes via a nozzle. The temperature during mixing was 30-40°C. After dosing, the product (MGDA-Na3 content: 67wt%) was homogenized for 2 more minutes, before discharging the wet solid product.

13 kg of the wet product was dried in a 16 L fluid bed drier during 8 minutes (air temperature 150°C; product end temperature 90°C).

Subsequently the product was milled using an Alexanderwerk friction sieve.

A free-flowing co granule product was obtained showing an MGDA-Na3 content of 74wt% and 4 wt% of silicate. The crystallinity of the product was 78%, showing solely the type I variety.

68wt% of the product showed a particle size in between 0.5 and 1.5 mm. The bulk density was 840 Kg/m³.

### Example 11 (according to the invention)

A mixture of a crumbly phase of 13.6 kg of solid MGDA-Na3, containing solely the type I crystal variety (MGDA-Na3 content 81wt%, crystallinity 75%) and 1.5 kg of a 40.7 wt% MGDA-Na3 aqueous solution were added to a 50 L Lödige Ploughshare mixer, operated at a rotation speed of 170 rpm, and subsequently 2.4 kg solid sodium silicate penta hydrate was dosed. The temperature during mixing was 30-40°C. After dosing, the reaction mixture was homogenized for 10 more minutes, before discharging the wet solid product.

13 kg of the wet product was dried in a 16 L fluid bed drier during 8 minutes (air temperature 150°C; product end temperature 90°C).

Subsequently the product was milled using an Alexanderwerk friction sieve.

A free-flowing product was obtained showing an MGDA-Na3 content of 74 wt%. The crystallinity of the product was 75%, showing solely the type I variety.

The bulk density was 780 Kg/m³.

### Example 12 (according to the invention)

15 kg of the dried Si/MGDA co-granule obtained in Example 11 containing approximately 4 wt% Silicate was re-dosed to a 50 L Lödige Ploughshare mixer, operated at a rotation speed of 170 rpm, and subsequently 1.5 kg of a 40.7 wt% MGDA-Na3 aqueous solution was added via a nozzle. Additionally, 113 grams of (≥27 %) sodium silicate solution was dosed via a separate nozzle onto the mixture. The temperature during mixing was 30-40°C. After dosing, the mixture was homogenized for 10 more minutes, before discharging the wet solid product. 1 kg of wet product was dried in a fluid bed dryer (5 L table dryer), at an air temperature of 90°C during 20 minutes.

Subsequently the product was milled using an Alexanderwerk friction sieve.

A free-flowing crystalline MGDA and silicate co-granule product was obtained. 73 wt% of the product showed a particle size in between 0.2 and 1.5 mm.

## Claims

1. Process to prepare solid crystalline co granules of methylglycine- N,N-diacetic acid (MGDA)-Naₓ, x being 2.5-3, comprising a step of drying a MGDA-Naₓ-containing crumbly phase composition containing on total weight of the crumbly phase composition:
(i) 70 - 87 wt.% of organic compounds and salts thereof containing 85 to 100 wt.% of MGDA-Na₃ on total organic compounds and salts thereof, wherein at least 60 wt.% of the MGDA-Na₃ is crystalline; and,
(ii) 13 - 30 wt.% of water;
in the presence of a second composition that contains at least one second component selected from the group of scale inhibitors, crystal inhibitors, film or spot preventing polymers, glass-corrosion inhibiting agents, pH modifiers, chelating agents, builders, bleaching agents, and surfactants.

2. Process of claim 1, wherein the crumbly phase composition contains on total weight of the composition:
(i) 70 - 80 wt.% of organic compounds and salts thereof containing 85 to 100 wt.% of MGDA-Naₓ on total organic compounds and salts thereof, wherein at least 60 wt.% of the MGDA-Naₓ is crystalline, and
(ii) 20 - 30 wt.% of water.

3. Process of claim 1 or claim 2, wherein the crumbly phase composition contains 75-80 wt.% of organic compounds and salts thereof on the basis of the total weight of the composition.

4. Process of any one of claims 1 to 3, wherein the organic compounds and salts thereof in the crumbly phase composition contain more than 90 wt.% of MGDA-Naₓ on the basis of total organic compounds.

5. Process of any one of claims 1 to 4, wherein the MGDA-Naₓ that is crystalline is at least 75% of crystal type I, preferably at least 90% on the basis of total crystalline MGDA-Naₓ content.

6. Process of any one of claims 1 to 5, wherein the MGDA is present between 50 and 80% in the L-enantiomeric form and between 20 and 50% in the D-enantiomeric form.

7. Process of any one of claims 1 to 6, wherein the second composition is a solid composition or an aqueous composition, preferably a saturated or supersaturated aqueous composition, preferably such that the obtained product mixture before the drying step remains in the crumble state.

8. Process of any one of claims 1 to 7, wherein the second composition is dosed to the crumbly phase in several steps or continuously over a period of time.

9. Process of any one of claims 1 to 8, wherein the second composition is added during the formation of the crumbly phase composition of MGDA-Naₓ.

10. Process of any one of claims 1 to 9, wherein the process involves as a drying step a step selected from the group of an evaporation step, a step of fluid bed drying, a step of thin film drying, a step of drum drying, and, a spray granulation step.

11. Process of any one of claims 1 to 10, wherein in the process a part of the crystalline product as obtained is recycled into the process and mixed with at least one of an aqueous composition containing MGDA-Naₓ, additional crumbly phase of MGDA-Naₓ and additional second composition.

12. Process of any one of claims 1 to 11, wherein the process is performed continuously.

13. Process of any one of claims 1 to 12, wherein the MGDA-Naₓ-containing crumbly phase composition is prepared by a process comprising mixing solid MGDA-Na₃ with a lesser amount of an aqueous composition of MGDA-Na₃ so that a crumbly phase is obtained **characterized by** weakly agglomerated MGDA-Na₃ particles covered with a layer of the aqueous composition, the majority of said MGDA-Na₃ particles comprising MGDA-Na₃ in a crystalline state, and the crumbly phase having a crumby texture and exhibiting rheological behavior that approximates dry particulate material.

14. Solid crystalline co granule MGDA-Naₓ product obtainable by the process of any one of claims 1 to 13 that contains on total co granule weight between 40 and 89 wt.% of MGDA-Naₓ and between 0.5 and 40 wt.% of the at least one second component.

15. The co-granule MGDA-Naₓ product of claim 14 that contains 90-99% crystals of the crystal type I and 1-10% crystals of crystal type III on the basis of the total amount of MGDA-Naₓ crystals,
wherein the MGDA is present between 50 and 80% in the L-enantiomeric form and between 20 and 50% in the D-enantiomeric form.

## Patentansprüche

1. Verfahren zur Herstellung fester kristalliner Co-Granulate von Methylglycin-N,N-diessigsäure (MGDA)-Naₓ, wobei x 2,5-3 beträgt, umfassend einen Schritt des Trocknens einer (MGDA)-Naₓ enthaltenden Krümelphasenzusammensetzung, die, auf das Gesamtgewicht der Krümelphasenzusammensetzung bezogen, Folgendes enthält:
(i) 70 - 87 Gew.-% organische Verbindungen und Salze davon, die 85 bis 100 Gew.-% MGDA-Na₃, auf die gesamten organischen Verbindungen und Salze davon bezogen, enthalten, wobei mindestens 60 Gew.-% der MGDA-Na₃ kristallin sind; und
(ii) 13 - 30 Gew.-% Wasser;
in Gegenwart einer zweiten Zusammensetzung, die mindestens eine zweite Komponente enthält ausgewählte aus der Gruppe von Wasserenthärtern, Kristallinhibitoren, Film- oder Fleckenverhinderungspolymeren, Glaskorrosionsverhinderungsmitteln, pH-Modifiziermitteln, Chelatbildnern, Gerüststoffen, Bleichmitteln und Tensiden.

2. Verfahren nach Anspruch 1, wobei die Krümelphasenzusammensetzung, auf das Gesamtgewicht der Zusammensetzung bezogen, Folgendes enthält:
(i) 70 - 80 Gew.-% organische Verbindungen und Salze davon, die 85 bis 100 Gew.-% MGDA-Naₓ, auf die gesamten organischen Verbindungen und Salze davon bezogen, enthalten, wobei mindestens 60 Gew.-% der MGDA-Naₓ kristallin sind und
(ii) 20 - 30 Gew.-% Wasser.

3. Verfahren nach Anspruch 1 oder Anspruch 2, wobei die Krümelphasenzusammensetzung 75-80 Gew.-% organische Verbindungen und Salze davon, auf der Basis des Gesamtgewichts der Zusammensetzung, enthält.

4. Verfahren nach einem der Ansprüche 1 bis 3, wobei die organischen Verbindungen und Salze davon in der Krümelphasenzusammensetzung mehr als 90 Gew.-% MGDA-Naₓ, auf der Basis der gesamten organischen Verbindungen, enthalten.

5. Verfahren nach einem der Ansprüche 1 bis 4, wobei die MGDA-Naₓ, die kristallin ist, zu mindestens 75 % vom Kristalltyp I, bevorzugt mindestens 90 %, auf der Basis des gesamten kristallinen MGDA-Naₓ-Gehalts, ist.

6. Verfahren nach einem der Ansprüche 1 bis 5, wobei die MGDA zu zwischen 50 und 80 % in der L-enantiomeren Form und zwischen 20 und 50 % in der D-enantiomeren Form vorliegt.

7. Verfahren nach einem der Ansprüche 1 bis 6, wobei die zweite Zusammensetzung eine feste Zusammensetzung oder eine wässrige Zusammensetzung, bevorzugt eine gesättigte oder übersättigte wässrige Zusammensetzung, bevorzugte derart ist, dass die erhaltene Produktmischung vor dem Trocknungsschritt im Krümelzustand bleibt.

8. Verfahren nach einem der Ansprüche 1 bis 7, wobei die zweite Zusammensetzung in mehreren Schritten oder kontinuierlich über eine Zeitspanne zu der Krümelphase dosiert wird.

9. Verfahren nach einem der Ansprüche 1 bis 8, wobei die zweite Zusammensetzung während der Bildung der Krümelphasenzusammensetzung von MGDA-Naₓ zugegeben wird.

10. Verfahren nach einem der Ansprüche 1 bis 9, wobei das Verfahren als Trocknungsschritt einen Schritt involviert, der aus der Gruppe eines Verdampfungsschritts, eines Schritts des Wirbelschichttrocknens, eines Schritts des Dünnschichttrocknens, eines Schritts des Trommeltrocknens und eines Sprühgranulierschritts ausgewählt wird.

11. Verfahren nach einem der Ansprüche 1 bis 10, wobei in dem Verfahren ein Teil des kristallinen Produkts wie erhalten in das Verfahren zurückgeführt und mit mindestens einer wässrigen Zusammensetzung, die MGDA-Naₓ, eine zusätzliche Krümelphase von MGDA-Naₓ und eine zusätzliche zweite Zusammensetzung enthält, gemischt wird.

12. Verfahren nach einem der Ansprüche 1 bis 11, wobei das Verfahren kontinuierlich ausgeführt wird.

13. Verfahren nach einem der Ansprüche 1 bis 12, wobei die MGDA-Naₓ enthaltende Krümelphasenzusammensetzung durch ein Verfahren hergestellt wird, das Mischen fester MGDA-Na₃ mit einer geringeren Menge einer wässrigen Zusammensetzung von MGDA-Na₃ umfasst, sodass eine Krümelphase erhalten wird, **gekennzeichnet durch** schwach angehäufte MGDA-Na₃-Partikel, die mit einer Schicht der wässrigen Zusammensetzung bedeckt sind, wobei die Mehrzahl der MGDA-Na₃-Partikel MGDA-Na₃ in einem kristallinen Zustand umfasst und die Krümelphase eine krümelige Konsistenz aufweist und ein rheologisches Verhalten aufzeigt, das sich trockenem teilchenförmigem Material annähert.

14. Festes kristallines Co-Granulat-MGDA-Naₓ-Produkt, das durch das Verfahren nach einem der Ansprüche 1 bis 13 erhältlich ist, das auf das gesamte Co-Granulatgewicht bezogen zwischen 40 und 89 Gew.-% MGDA-Naₓ und zwischen 0,5 und 40 Gew.-% mindestens einer zweiten Komponente enthält.

15. Co-Granulat-MGDA-Naₓ-Produkt nach Anspruch 14, das 90-99 % Kristalle des Kristalltyps I und 1-10 % Kristalle des Kristalltyps III, auf der Basis der Gesamtmenge von MGDA-Naₓ-Kristallen, enthält,
wobei die MGDA in einer Menge zwischen 50 und 80 % in der L-enantiomeren Form und zwischen 20 und 50 % in der D-enantiomeren Form vorliegt.

## Revendications

1. Procédé de préparation de co-granulés cristallins solides d'acide méthylglycine-N,N-diacétique (MGDA)-Naₓ, x étant compris entre 2,5 et 3, comprenant une étape de séchage d'une composition de phase friable contenant du MGDA-Naₓ contenant par rapport au poids total de la composition de phase friable :
(i) de 70 à 87 % en poids de composés organiques et de sel de ceux-ci contenant de 85 à 100 % en poids de MGDA-Na₃ par rapport au total des composés organiques et des sels de ceux-ci, dans lequel au moins 60 % en poids du MGDA-Na₃ est cristallin ; et
(ii) de 13 à 30 % en poids d'eau ;
en présence d'une deuxième composition contenant au moins un deuxième composant choisi dans le groupe des inhibiteurs de tartre, des inhibiteurs de cristaux, des polymères anti-taches ou anti-films, des agents inhibiteurs de corrosion du verre, des modificateurs de pH, des agents chélatants, des agents de construction, des agents de blanchiment et des tensioactifs.

2. Procédé selon la revendication 1, dans lequel la composition de phase friable contient par rapport au poids total de la composition :
(i) de 70 à 80 % en poids de composés organiques et de sel de ceux-ci contenant de 85 à 100 % en poids de MGDA-Naₓ par rapport au total des composés organiques et des sels de ceux-ci, dans lequel au moins 60 % en poids du MGDA-Naₓ est cristallin ; et
(ii) de 20 à 30 % en poids d'eau.

3. Procédé selon la revendication 1 ou la revendication 2, dans lequel la composition de phase friable contient de 75 à 80 % en poids de composés organiques et de sels de ceux-ci en se basant sur le poids total de la composition.

4. Procédé selon l'une quelconque des revendications 1 à 3, dans lequel les composés organiques et des sels de ceux-ci dans la composition de phase friable contiennent plus de 90 % en poids de MGDA-Naₓ, en se basant sur le total des composés organiques.

5. Procédé selon l'une quelconque des revendications 1 à 4, dans lequel le MGDA-Naₓ cristallin se compose d'au moins 75 % de cristaux de type I, de préférence d'au moins 90 % en se basant sur la teneur totale de MGDA-Naₓ cristallin.

6. Procédé selon l'une quelconque des revendications 1 à 5, dans lequel le MGDA est présent entre 50 et 80 % sous forme énantiomérique L et entre 20 et 50 % sous forme énantiomérique D.

7. Procédé selon l'une quelconque des revendications 1 à 6, dans lequel la deuxième composition est une composition solide ou une composition aqueuse, de préférence une composition aqueuse saturée ou sursaturée, de préférence de sorte que le mélange de produits obtenu avant l'étape de séchage reste à l'état friable.

8. Procédé selon l'une quelconque des revendications 1 à 7, dans lequel la seconde composition est ajoutée à la phase friable en plusieurs étapes ou de manière continue sur une période de temps.

9. Procédé selon l'une quelconque des revendications 1 à 8, dans lequel la seconde composition est ajoutée lors de la formation de la composition de phase friable de MGDA-Naₓ.

10. Procédé selon l'une quelconque des revendications 1 à 9, dans lequel le procédé implique comme étape de séchage une étape choisie dans le groupe d'une étape d'évaporation, d'une étape de séchage en lit fluidisé, d'une étape de séchage en film mince, d'une étape de séchage sur tambour et d'une étape de granulation par pulvérisation.

11. Procédé selon l'une quelconque des revendications 1 à 10, dans lequel dans le procédé une partie du produit cristallin tel qu'obtenu est recyclée dans le procédé et mélangée à au moins une parmi une composition aqueuse contenant du MGDA-Naₓ, une phase friable supplémentaire de MGDA-Naₓ et une deuxième composition supplémentaire.

12. Procédé selon l'une quelconque des revendications 1 à 11, dans lequel le procédé est effectué en continu.

13. Procédé selon l'une quelconque des revendications 1 à 12, dans lequel la composition de phase friable contenant du MGDA-Na₃ est préparée par un procédé comprenant le mélange de MGDA-Na₃ solide avec une quantité moindre d'une composition aqueuse de MGDA-Na₃ de sorte à obtenir une phase friable **caractérisée par** des particules de MGDA-Na₃ faiblement agglomérées recouvertes d'une couche de la composition aqueuse, la majorité desdites particules de MGDA-Na₃ comprenant du MGDA-Na₃ à l'état cristallin, et la phase friable ayant une texture friable et présentant un comportement rhéologique proche de celui d'un matériau particulaire sec.

14. Produit de MGDA-Naₓ co-granulé cristallin solide pouvant être obtenu par le procédé selon l'une quelconque des revendications 1 à 13 contenant par rapport au poids total de co-granulés entre 40 et 89 % en poids de MGDA-Naₓ et entre 0,5 et 40 % en poids de l'au moins un deuxième composant.

15. Produit de MGDA-Naₓ co-granulé selon la revendication 14 contenant de 90 à 99 % de cristaux de type cristallin I et de 1 à 10 % de cristaux de type cristallin III en se basant sur la quantité totale de cristaux de MGDA-Naₓ,
dans lequel le MGDA est présent entre 50 et 80 % sous forme énantiomérique L et entre 20 et 50 % sous forme énantiomérique D.
